# EUROPEAN PATENT SPECIFICATION

(11) **EP 3 886 610 B1**
(45) Date of publication and mention of the grant of the patent: **11.09.2024**
(21) Application number: 19798249.9
(22) Date of filing: 01.11.2019
(51) Int. Cl.: A23L 33/00, A23L 33/10, A61K 31/702

(54) **INFANT NUTRITIONAL COMPOSITION FOR USE IN THE ENHANCEMENT OF PANCREATIC MATURATION AND INSULIN BIOSYNTHESIS**
SÄUGLINGSERNÄHRUNGSZUSAMMENSETZUNG ZUR VERWENDUNG BEI DER VERBESSERUNG DER PANKREASREIFUNG UND DER INSULINBIOSYNTHESE
COMPOSITION NUTRITIONNELLE POUR NOURRISSONS POUR UNE UTILISATION DANS L'AMÉLIORATION DE LA MATURATION PANCRÉATIQUE ET DE LA BIOSYNTHÈSE D'INSULINE

(30) Priority: 30.11.2018 EP 18209519
(43) Date of publication of application: 06.10.2021
(73) Proprietor: Société des Produits Nestlé S.A., 1800 Vevey (CH)
(72) Inventor: GARCIA-RODENAS, Clara,Lucia, 1072 FOREL (CH); RAMOS NIEVES, José, Manuel, 1004 LAUSANNE (CH)
(74) Representative: Loiseau, François Michel
(86) International application number: PCT/EP2019/079962
(87) International publication number: WO 2020/108915

(56) References cited:
- WO-A1-2017/129639
- WO-A1-2017/129650
- WO-A1-2018/215573
- US-A1- 2016 296 543
- US-A1- 2016 296 585
- US-A1- 2017 295 838
- US-A1- 2018 104 267
- US-A1- 2018 333 426
- US-B2- 6 946 451
- US-B2- 9 636 367
- S. BLAT ET AL: "Does early oligosaccahrides consumption affect pancreas maturation", JOURNAL OF DEVELOPMENTAL ORIGINS OF HEALTH AND DISEASE, vol. 6, no. S1, 1 January 2015 (2015-01-01), pages S13, XP055564034
- STOLOVICH-RAIN MIRI ET AL: "Weaning Triggers a Maturation Step of Pancreatic [beta] C", DEVELOPMENTAL CELL, CELL PRESS, US, vol. 32, no. 5, 5 February 2015 (2015-02-05), pages 535 - 545, XP029144916, ISSN: 1534-5807, DOI: 10.1016/J.DEVCEL.2015.01.002
- SUSAN BONNER-WEIR ET AL: "Dynamic development of the pancreas from birth to adulthood", UPSALA JOURNAL OF MEDICAL SCIENCES., vol. 121, no. 2, 21 March 2016 (2016-03-21), SE, pages 155 - 158, XP055564061, ISSN: 0300-9734, DOI: 10.3109/03009734.2016.1154906

## Description

### Field of the invention

The present invention relates to a nutritional composition comprising at least one human milk oligosaccharide (HMO) for use in enhancing the pancreatic development and/or pancreatic maturation of infants. The invention also relates to the improvement of glucose management and to ultimately help preventing and/or improving related health disorders (such as obesity or type 2 diabetes) in infants, young children or individuals later in life.

### Background of the invention

The optimal development of functional organs is of ultimate importance for all infants and especially for infants which are at risk because their overall development is impacted by some genetic factors, external factors, infections, metabolic disorders, illness, or suboptimal health conditions. Such development start evidently *in-utero* but also continues after birth. Hence the nutritional status of the infant is both of critical importance *in-utero* (as it can be influenced to the health status of the mother, affecting the nutrients supply via the umbilical cord) and it also of importance after birth. In the early days/months the nutritional status depends of the nutrient supply provided (Breast Feeding, Infant formula) but also depends on the ability of the infant to ingest (suckling capacity), digest (gastro-intestinal functions), absorb and utilize the nutrients.

The pancreas produces various enzymes that are secreted into the small intestine where they contribute to digestive functions. The pancreas also produces various hormones, and in particular insulin that it is secreted into the bloodstream, where it regulates the body's glucose or sugar level. As such the pancreas participates in at least 2 extremely important metabolic function: The intestinal digestive function and glucose management. The dysregulation of one or both of these functions has direct effect on the health status of the individual, both at the time of dysfunction and later in life. For example nutrient malabsorption by the intestine can induce growth retardation, poor neurodevelopment but also affect immune defences at the short terms and later in life. Low insulin biosynthesis by the pancreas can induce high circulating glucose levels and reduced glucose availability to organs as important as brain or muscle immediately but also metabolic disorders such as diabetes or overweight/obesity later in life.

Pancreatic development starts *in-utero* but continues along the infancy, childhood and adulthood. As explained by Susan Bonner-Weir et al (Dynamic development of the pancreas from birth to adulthood; Ups J Med Sci 2016 May; 121(2):155-158, PMID:260088'6), after birth the endocrine pancreas continues its development, a complex process that involves both the maturation of islet cells and a marked expansion of their numbers. New beta cells are formed both by duplication of pre-existing cells and by new differentiation (neogenesis) across the first postnatal weeks, with the result of beta cells of different stages of maturation even after weaning.

Consequently there is a need to insure the most adequate pancreatic development and maturation in all infants and over a long duration. More specifically there is a need to insure the optimal pancreatic development and maturation for infants who are particularly at risk, like infants born preterm (hence not having completed their expected pancreatic development in-utero), fragile infants (small for gestational age, low birth weight infants, very-low or extremely-low birth weight infants), infants suffering from weak health status, infections or diseases, infant having particular genetic predisposition or infants born from mother having not insured optimal in-utero development to their infant because of their weak health status.

The insulin biosynthesis (production) and insulin secretion by the pancreas is known to be affected by several factors, such as genetic factors, plasma levels of glucose or some peptides such as Ghrelin (see "Ghrelin, A New Gastrointestinal Endocrine Peptide that Stimulates Insulin Secretion: Enteric Distribution, Ontogeny, Influence of Endocrine, and Dietary Manipulations", Heung-Man Lee Guiyun Wang Ella et al, Endocrinology, 43-1, pages 185-190; 1-1-2012).

The biosynthesis and secretion of insulin by the pancreas can also be directly or indirectly influenced by the subject's nutrition (for example the ingestion of sugars) - but these aspects are much less known.

In particular the secretion of insulin has been described to be influenced by nutritional composition which can be in the form of a modified powder milk for infants containing di- or oligosaccharides like 2FL (EP1332759A / US6946451B2; Kyowa Hakko Kogyo Co. LTD, published on 6.8.2003),

Similarly, US9636367B2 discloses a method for increasing insulin sensitivity and/or reducing insulin resistance in an infant who is predisposed for developing insulin resistance, the method comprising administering at least one LC-PUFA, at least one probiotic and an oligosaccharide mixture containing 2FL, LNnT and 3SL or 6SL.

Importantly indeed the secretion of insulin by the pancreatic β-cells and the capacity of the pancreatic (islets) to produce insulin are regulated by two different pathways. Insulin biosynthesis is regulated by glucose and other nutrient levels as well as by effectors both at transcriptional and translational levels. Insulin secretion is regulated mainly by glucose levels and modulated by other nutrients and hormonal cues. Insulin secretion is generated by cellular signalling transduction pathways as well as by the fusion/transport/docking of the insulin granules (Regulation of Insulin Synthesis and Secretion and Pancreatic Beta-Cell Dysfunction in Diabetes, Zhuo Fu, E. R. Gilbert and Dongmin Lui, Curr. Diabetes Rev. 2013, Jan 1; 9(1):25-53).

Some of the effectors having an action on the secretion of insulin from the pancreas may indeed be different from those acting on the biosynthesis of the insulin by the pancreatic cells. Those effectors may act via different mechanisms than those involved in the biosynthesis of insulin by pancreatic cells. Similarly enhancing the biosynthesis of insulin by the pancreatic islets may or may not boost the secretion as the pancreas also acts as a reservoir for insulin. However, without being bound by the theory, it is thought that the enhancement of the insulin biosynthesis has an effect on insulin secretion capacity into the blood stream over time during the life cycle. On contrary, the secretion mechanisms mainly deal with the short-term capacity to increase blood insulin concentrations, until the storage pancreatic capacity is exhausted. In simple words, they empty the reservoir but do not allow for its replenishment. Being mechanistically different pathways, one effector on secretion is not necessarily an effector on biosynthesis of insulin by the pancreatic cells.

Accordingly it is of importance to promote the pancreatic development and/or maturation in the early days, preferably in a manner that is non-invasive (on the other metabolic pathways), mild and does not induce or minimize side effects. Without being bound by the theory, it is thought that such promotion of the pancreatic development and/or maturation of the pancreas can interplay at least at 3 levels: The increase of the number of pancreatic β-cells, the maturation of the individual cells and/or the increase of the insulin synthesis amount per cell. All three pathways have the potentiality to induce the desired overall boost of the insulin secretion and thus also have a long term influence on the glucose management of the individuals.

In that context, nutritional interventions are a tool of choice for such optimization of the health conditions.

There is a need to identify and make use of especially targeted nutritional ingredients or nutritional compositions to induce an enhanced or optimal development or maturation of the pancreas and/or of the pancreatic function.

There is a need to deliver such effect at the earliest possible stage in infancy and preferably to observe the benefits of such nutritional intervention also later in life.

There is a need to boost the biosynthesis of insulin by the pancreatic islets in infants, and enhance the levels of insulin biosynthesis.

There is a need to influence the glucose management at the time of nutritional intervention and later in life and reduce the risk, prevent and/or treat of associated metabolic disorders such as diabetes (type 2), obesity or overweight.

There is a particular need to induce these beneficial effects in infants affected or at risk to be affected by sub-optimal pancreatic development or maturation.

There is a particular need to induce these beneficial effects in fragile, preterm, low-birth-weight, very- or extremely-low-birth-weight infants or infants having particular risk (including genetic risk) of under-maturation of the pancreas or predisposition to be affected by suboptimal glucose management (or of the associated diseases).

There is a need to deliver such beneficial health effects in a manner that does not unbalance other metabolic or development pathways and/or does not induce undesired side effects.

There is a need to deliver such health benefits via enteral nutrition in a manner that is able to resist the passage through the gastro-intestinal tract.

There is a need to bring the pancreatic development and/or maturation and/or the level of related pancreatic insulin biosynthesis of infants back into the normality zone whenever they deviate from normal development.

Mother's milk and breast feeding is best and is recommended for all infants. However, in some cases breast feeding is inadequate or unsuccessful for medical reasons or the mother chooses not to breast feed. Infant formulae have been developed for these situations. Fortifiers have also been developed to enrich mother's milk or infant formula with specific ingredients.

There is clearly a need for developing nutritional compositions for use in enhancing the pancreatic development and/or maturation and/or in increasing / enhancing the level of insulin biosynthesis / production by the pancreas.

There is also a need to deliver such health benefits in a manner that is particularly suitable for the young subjects (infants and young children), in a manner that does not involve a classical pharmaceutical intervention as these infants or young children are particularly fragile. There is a need to deliver such health benefits in these infants or young children in a manner that does not induce side effects and/or in a manner that is easy to deliver, and well accepted by the parents or health care practitioners.

There is also a need to deliver such benefits in a manner that does keep the cost of such delivery reasonable and affordable by most. There is a need to deliver the targeted benefits and compositions in a manner that is convenient to use and to which the subjects will adhere easily.

### Summary of the invention

The present inventors have found that nutritional compositions comprising at least one fucosylated oligosaccharide (preferably 2FL) can advantageously be used to promote and/or enhance the development and/or maturation of the pancreas in young children or infants, wherein said young children or infants are subject in need and/or are at risk of under-development of pancreas and/or are born preterm and/or suffering from intra-uterine-growth retardation (IUGR) and/or born with low birth weight (LBW), very-low-birth-weight (VLBW), or extremely-low-birth-weight (ELBW). In one aspect, not pertaining to the invention, the composition promotes / increase / enhances the pancreatic biosynthesis / production of the insulin by the pancreatic cells. In one aspect the composition is an infant formula, a supplement or a human milk fortifier, and is particularly beneficial to fragile or preterm infants. In one aspect the composition of the invention also comprises other oligosaccharides, such as LnNT.

### Brief description of the figures

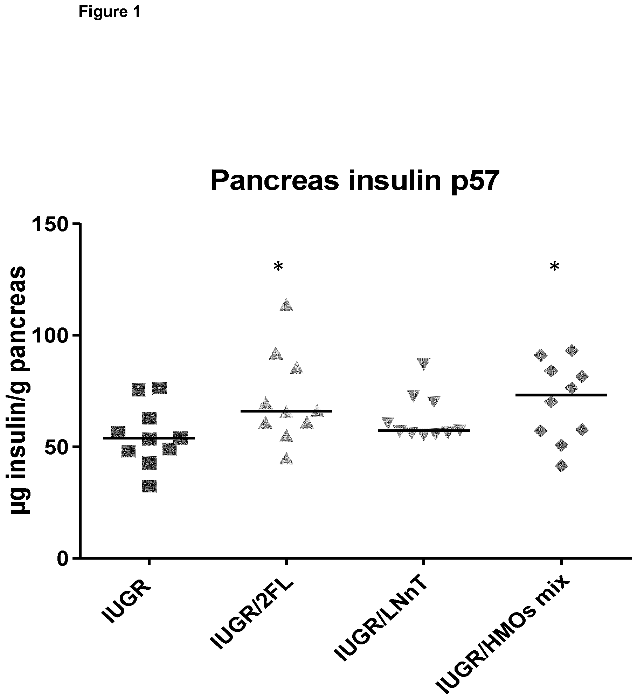

**Figure 1** represents the concentration of insulin in pancreas of rats at postnatal day 57, in various groups :
   - IUGR rats: IUGR (intrauterine growth restricted) rats; gavaged with 3 g/kg/BW of maltodextrin from d7 to d21 and fed with a control diet from d22 to d57. IUGR rats/2FL: IUGR rats gavaged with 3 g/kg/BW of 2FL from d7 to d21 and fed with a diet supplemented with 4.5 wt% human milkoligosaccharide 2FL from d22 to d57.
   - IUGR rats/LNnT: IUGR rats gavaged with 3 g/kg/BW of LNnT from d7 to d21 and fed with a diet supplemented with 4.5 wt% human milk oligosaccharide LNnT from d22 to d57.
   - IUGR rats/HMOs mix: IUGR rats gavaged with 3 g/kg/BW of HMOs mix from d7 to d21 and fed with a diet supplemented with 4.5 wt% human milk oligosaccharides (2FL+LNnT in a weight ratio of 2:1) from d22 to d57.
      Indication (*) means *P< 0.05

### Detailed description of the invention

### Definitions:

As used herein, the following terms have the following meanings.

The term **"infant"** means a child under the age of 12 months. The expression " **child**" means a child aged between one and three years, also called toddler. The expression "**child**" means a child between three and seven years of age.

An "**infant or young child born by C-section**" means an infant or young child who was delivered by caesarean. It means that the infant or young child was not vaginally delivered.

An "**infant or young child vaginally born**" means an infant or young child who was vaginally delivered and not delivered by caesarean.

A "**preterm" or "premature**" means an infant or young child who was not born at term. Generally it refers to an infant or young child born prior 37 weeks of gestation.

An "**infant having a low birth weight**" means a new born having a body weight below 2500g (5.5 pounds) either because of preterm birth or restricted fetal growth. It therefore encompasses:
- infant or young child who has/had a body weight from 1500 to 2500 g at birth (usually called "low birth weight" or LBW)
- infant or young child who has/had a body weight from 1000 to 1500 g at birth (called "very low birth weight" or VLBW)
- infant or young child who has/had a body weight under 1000 g at birth (called "extremely low birth weight" or ELBW).

An "**infant born small for gestational age (SGA)**" means a baby with birth weights below the 10^{th} percentile for babies of the same gestational age.

The expression "**nutritional composition**" means a composition which nourishes a subject. This nutritional composition is usually to be taken orally. In some cases it can be provided intravenously. It usually includes a lipid or fat source and a protein source. The nutritional composition is intended to be administrated to subjects.

The nutritional composition of the invention is man-made, .i.e. it can be for example formulated by humans from ingredients (naturally-occurring, biological or chemical compounds) or it can be for example derived from biological fluids but processed by a human intervention. The nutritional composition of the invention is not human breast milk. In a particular embodiment the nutritional composition of the present invention is a "synthetic nutritional composition". The expression "**synthetic nutritional composition**" means a mixture obtained by chemical and/or biological means, which can be chemically similar or identical to the mixture naturally occurring in mammalian milks (i.e. the synthetic composition is not human breast milk).

In a particular embodiment the composition of the present invention is a hypoallergenic nutritional composition. The expression "**hypoallergenic nutritional composition**" means a nutritional composition which is unlikely to cause allergic reactions.

The expression "**infant formula**" as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula".

A "**follow-up formula**" or "**follow-on formula**" is given from the 6th month onwards. It constitutes the principal liquid element in the progressively diversified diet of this category of person.

The expression "**baby food**" means a foodstuff intended for particular nutritional use by infants or children during the first years of life.

The expression "**infant cereal composition**" means a foodstuff intended for particular nutritional use by infants or young children during the first years of life.

The expression "**growing**-**up milk**" (or GUM) refers to a milk-based drink generally with added vitamins and minerals, that is intended for young children, as of one year of age and usually up to the third year of age, or children up to the seventh year of age.

The term "**fortifier**" refers to liquid or solid nutritional compositions suitable for mixing with breast milk or infant formula.

The expression "**weaning period**" means the period during which the mother's milk is substituted by other food in the diet of an infant or young child.

The expressions "**days/weeks/months/years of life**" and "**days/weeks/months/years of birth**" can be used interchangeably.

The "**mother's milk**" should be understood as the breast milk or the colostrum of the mother.

An "**oligosaccharide**" is a saccharide polymer containing a small number (typically three to ten) of simple sugars (monosaccharides).

The term "**HMO**" or "**HMOs**" refers to **Human Milk Oligosaccharide(s).** These carbohydrates are highly resistant to enzymatic hydrolysis, indicating that they may display essential functions not directly related to their caloric value. It has especially been illustrated that they play a vital role in the early development of infants and young children, such as the maturation of the immune system. Many different kinds of HMOs are found in the human milk. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk - over 130 such structures have been identified so far. Almost all of them have a lactose moiety at their reducing end while sialic acid and/or fucose (when present) occupy terminal positions at the nonreducing ends. The HMOs can be acidic (e.g. charged sialic acid containing oligosaccharide) or neutral (e.g. fucosylated oligosaccharide).

A "**fucosylated oligosaccharide**" is an oligosaccharide having a fucose residue. It has a neutral nature. Some examples are 2-FL (2'-fucosyllactose), 3-FL (3-fucosyllactose), difucosyllactose, lacto-N-fucopentaose (e.g. lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose, difucosyllacto-N-hexaose I, difucosyllacto-N-neohexaose II and any combination thereof. Without wishing to be bound by theory it is believed that the fucosyl-epitope of the fucosylated oligosaccharides may act as decoy at the mucosal surface. By a competition effect, it may prevent and/or limit the action of the pathogens responsible of infections (of viral or bacterial origin) or of their secreted components (e.g. toxins), especially by avoiding their binding to natural ligands, and without to be bound by theory, this is believed to therefore reduce the risk of infections/inflammations, and particularly the risk of LRT/ear infections and/or inflammations. In addition, the fucosylated oligosaccharides are thought to boost growth and metabolic activity of specific commensal microbes reducing inflammatory response and creating an environment unfavourable for pathogens thus leading to colonization resistance.

The expressions "**fucosylated oligosaccharides comprising a 2'-fucosyl-epitope**" and "**2-fucosylated oligosaccharides**" encompass fucosylated oligosaccharides with a certain homology of form since they contain a 2'-fucosyl-epitope, therefore a certain homology of function can be expected. Without wishing to be bound by theory the 2'-fucosyl-epitope of these fucosylated oligosaccharides is believed to be particularly specific to pathogens (or their secreted components) involved in the LRT and/or ear infections. The terms 2FL, 2'FL, 2-FL, 2'-FL, 2-fucosyllactose and 2'-fucosyllactose are used interchangeably with the same meaning.

The expression "**N-acetylated oligosaccharide(s)**" encompasses both "N-acetyl-lactosamine" and "oligosaccharide(s) containing N-acetyl-lactosamine". They are neutral oligosaccharides having an N-acetyl-lactosamine residue. Suitable examples are LNT (lacto-N-tetraose), para-lacto-N-neohexaose (para-LNnH), LNnT (lacto-N-neotetraose), disialyllacto-N-tetraose (DSLNT) and any combinations thereof. Other examples are lacto-N-hexaose, lacto-N-neohexaose, para- lacto-N-hexaose, para-lacto-N-neohexaose, lacto-N-octaose, lacto-N- neooctaose, iso- lacto-N-octaose, para- lacto-N-octaose and lacto-N-decaose.

The expression "at least one fucosylated oligosaccharide" and "at least one N-acetylated oligosaccharide" means "at least one type of fucosylated oligosaccharide" and "at least one type of N-acetylated oligosaccharide".

A "**precursor of HMO**" is a key compound that intervenes in the manufacture of HMO, such as sialic acid and/or fucose.

A "**sialylated oligosaccharide**" is a charged sialic acid containing oligosaccharide, i.e. an oligosaccharide having a sialic acid residue. It has an acidic nature. Some examples are 3-SL (3' sialyllactose) and 6-SL (6' sialyllactose).

The term "**prebiotic**" means non-digestible carbohydrates that beneficially affect the host by selectively stimulating the growth and/or the activity of healthy bacteria such as bifidobacteria in the colon of humans *(*Gibson GR, Roberfroid MB. Dietary modulation of the human colonic microbiota: introducing the concept of prebiotics. J Nutr. 1995;125:1401-12*).*

The term "**probiotic**" means microbial cell preparations or components of microbial cells with a beneficial effect on the health or well-being of the host. (Salminen S, Ouwehand A. Benno Y. et al. "Probiotics: how should they be defined" Trends Food Sci. Technol. 1999:10 107-10). The microbial cells are generally bacteria or yeasts. Probiotics can be in a live form (replicating) or in a non-replicating form.

The term "**cfu**" should be understood as colony-forming unit. All percentages are by weight unless otherwise stated.

In addition, in the context of the invention, the terms "comprising" or "comprises" do not exclude other possible elements. The composition of the present invention, including the many embodiments described herein, can comprise, consist of, or consist essentially of the essential elements and limitations of the invention described herein, as well as any additional or optional ingredients, components, or limitations described herein or otherwise depending on the needs.

### Form of the composition:

The nutritional composition of the present invention can be in solid form (e.g. powder) or in liquid form. The amount of the various ingredients (e.g. the oligosaccharides) can be expressed in g/100g of composition on a dry weight basis when it is in a solid form, e.g. a powder, or as a concentration in g/L of the composition when it refers to a liquid form (this latter also encompasses liquid composition that may be obtained from a powder after reconstitution in a liquid such as milk, water..., e.g. a reconstituted infant formula or a follow-on/follow-up formula or a growing-up milk or an infant cereal product or any other formulation designed for infant nutrition). When g/L are used in reference to a powder (dry) product, the g/L amount refers to the amount present in the liquid form after reconstitution into a ready-to-use product, in a manner suitable to the intended use (i.e. according to the instructions).

The nutritional composition according to the invention can be for example an infant formula, a starter infant formula, a follow-on or follow-up formula, a fortifier such as a human milk fortifier, or a supplement. In an embodiment the nutritional composition of the invention is a growing-up milk. Less preferably the invention could be also applied to a baby food and an infant cereal composition.

In one embodiment the nutritional composition of the invention is a complete nutritional composition (fulfilling all or most of the nutritional needs of the subject). In another embodiment the nutrition composition is a supplement or a fortifier intended for example to supplement human milk or to supplement an infant formula or a follow-on/follow-up formula.

In some particular embodiments, the composition of the invention is an infant formula, a fortifier or a supplement that may be intended for the first 4, 6 or 12 months of age. In a preferred embodiment the nutritional composition of the invention is an infant formula. It is indeed believed that the nutritional intervention of the invention may be most effective when enacted at an early stage of life (for example the first 1, 4, 6, 12 months of age), as it has greater impact on the pancreatic development and maturation and thus on the insulin synthesis and glucose management, especially later in life.

In some other embodiments the nutritional composition of the present invention is a fortifier. The fortifier can be a breast milk fortifier (e.g. a human milk fortifier) or a formula fortifier such as an infant formula fortifier or a follow-on/follow-up formula fortifier.

When the nutritional composition is a supplement, it can be provided in the form of unit doses. In such cases it is particularly useful to define the amount of the subject oligosaccharides and optionally other oligosaccharides in terms or daily dose to be administered to the infant or young child, such as described above.

The nutritional composition of the present invention can be in solid (e.g. powder), liquid or gelatinous form.

In a specific embodiment the nutritional composition is a supplement in powder form and provided in a sachet, or in the form of a syrup. When the supplement is in powder form, it may comprise a carrier. It is however preferred that the supplement is devoid of a carrier. When the supplement is in the form of a syrup, the HMOs are preferably dissolved or suspended in water acidified with citrate.

### Supplement

In another embodiment, the composition of the invention may be a supplement. The supplement may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, the supplement may contain an organic or inorganic carrier material suitable for oral or parenteral administration as well as vitamins, minerals trace elements and other micronutrients in accordance with the recommendations of Government bodies such as the USRDA.

### Administration regimen of the composition

In some embodiments the composition according to the invention can be for use before and/or during the weaning period.

The nutritional composition can be administered (or given or fed) at an age and for a period that depends on the needs.

The nutritional composition can be for example given immediately after birth of the infants. The composition of the invention can also be given during the first week of life of the infant, or during the first 2 weeks of life, or during the first 3 weeks of life, or during the first month of life, or during the first 2 months of life, or during the first 3 months of life, or during the first 4 months of life, or during the first 6 months of life, or during the first 8 months of life, or during the first 10 months of life, or during the first year of life, or during the first two years of life or even more. In some particularly advantageous embodiments of the invention, the nutritional composition is given (or administered) to an infant within the first 4, 6 or 12 months of birth of said infant. In some other embodiments, the nutritional composition of the invention is given few days (e.g. 1, 2, 3, 5, 10, 15, 20...), or few weeks (e.g. 1, 2, 3, 4, 5, 6, 7, 8, 9, 10...), or few months (e.g. 1, 2, 3, 4, 5, 6, 7, 8, 9, 10...) after birth. This may be especially the case when the infant is premature, but not necessarily.

In one embodiment the composition of the invention is given to the infant or young child as a supplementary composition to the mother's milk. In some embodiments the infant or young child receives the mother's milk during at least the first 2 weeks, first 1, 2, 4, or 6 months. In one embodiment the nutritional composition of the invention is given to the infant or young child after such period of mother's nutrition, or is given together with such period of mother's milk nutrition. In another embodiment the composition is given to the infant or young child as the sole or primary nutritional composition during at least one period of time, e.g. after the 1^{st}, 2^{nd} or 4^{th} month of life, during at least 1, 2, 4 or 6 months.

### Other ingredients

### Probiotics

The nutritional composition of the present invention can further comprise at least one probiotic (or probiotic strain), such as a probiotic bacterial strain.

The probiotic microorganisms most commonly used are principally bacteria and yeasts of the following genera: *Lactobacillus spp., Streptococcus spp., Enterococcus spp., Bifidobacterium spp.* and *Saccharomyces spp.*

In some particular embodiments, the probiotic is a probiotic bacterial strain. In some specific embodiments, it is particularly *Bifidobacteria* and/or *Lactobacilli.*

Suitable probiotic bacterial strains include *Lactobacillus rhamnosus* ATCC 53103 available from Valio Oy of Finland under the trademark LGG, *Lactobacillus rhamnosus* CGMCC 1.3724, *Lactobacillus paracasei* CNCM 1-2116, *Lactobacillus johnsonii* CNCM I-1225, *Streptococcus salivarius* DSM 13084 sold by BLIS Technologies Limited of New Zealand under the designation KI2, *Bifidobacterium lactis* CNCM 1-3446 sold *inter alia* by the Christian Hansen company of Denmark under the trademark Bb 12, *Bifidobacterium longum* ATCC BAA-999 sold by Morinaga Milk Industry Co. Ltd. of Japan under the trademark BB536, *Bifidobacterium breve* sold by Danisco under the trademark Bb-03, *Bifidobacterium breve* sold by Morinaga under the trade mark M-16V, *Bifidobacterium infantis* sold by Procter & Gamble Co. under the trademark Bifantis and *Bifidobacterium breve* sold by Institut Rosell (Lallemand) under the trademark R0070.

The nutritional composition according to the invention may contain from 10³ to 10¹² cfu of probiotic strain, more preferably between 10⁷ and 10¹² cfu such as between 10⁸ and 10¹⁰ cfu of probiotic strain per g of composition on a dry weight basis.

In one embodiment the probiotics are viable (i.e. live; i.e. able to replicate). In another embodiment the probiotics are non-replicating (i.e. inactivated). Probiotics may have been rendered non-replicating (inactivated) by any targeted treatment known in the art, and preferably such treatment comprising or consisting of a heat treatment. There may be both viable probiotics and inactivated probiotics in some other embodiments. Probiotic components and metabolites can also be added.

### Carbohydrate source

The nutritional composition according to the present invention generally contains a carbohydrate source. This is particularly preferable in the case where the nutritional composition of the invention is an infant formula. In this case, any carbohydrate source conventionally found in infant formulae such as lactose, sucrose, saccharose, maltodextrin, starch and mixtures thereof may be used although one of the preferred sources of carbohydrates is lactose.

### Lipid source

The nutritional composition according to the present invention generally contains a source of lipids. This is particularly relevant if the nutritional composition of the invention is an infant formula. In this case, the lipid source may be any lipid or fat which is suitable for use in infant formulae. Some suitable fat sources include palm oil, structured triglyceride oil, high oleic sunflower oil and high oleic safflower oil, medium-chain-triglyceride oil. The essential fatty acids linoleic and α-linolenic acid may also be added, as well small amounts of oils containing high quantities of preformed arachidonic acid and docosahexaenoic acid such as fish oils or microbial oils. The fat source may have a ratio of n-6 to n-3 fatty acids of about 5:1 to about 15:1; for example about 8:1 to about 10:1.

### Protein source

The nutritional composition according to the invention generally contains a protein source. In some embodiments, the protein can be in an amount of from 1.6 to 3 g per 100 kcal. In some embodiments, especially when the composition is intended for premature infants, the protein amount can be between 2.4 and 4 g/100kcal or more than 3.6 g/100kcal. In some other embodiments, especially when the composition is an infant formula, the protein amount can be below 2.0 g per 100 kcal, e.g. between 1.8 to 2 g/100kcal, or in an amount below 1.8g per 100 kcal.

The type of protein is not believed to be critical to the present invention provided that the minimum requirements for essential amino acid content are met and satisfactory growth is ensured. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in any desired proportions.

In some advantageous embodiments the protein source is whey predominant (i.e. more than 50% of proteins are coming from whey proteins, such as 60% or 70%).

The proteins may be intact or hydrolysed or a mixture of intact and hydrolysed proteins. By the term "intact" is meant that the main part of the proteins are intact, i.e. the molecular structure is not altered, for example at least 80% of the proteins are not altered, such as at least 85% of the proteins are not altered, preferably at least 90% of the proteins are not altered, even more preferably at least 95% of the proteins are not altered, such as at least 98% of the proteins are not altered. In a particular embodiment, 100% of the proteins are not altered.

The term "hydrolysed" means in the context of the present invention a protein which has been hydrolysed or broken down into its component amino acids. The proteins may be either fully or partially hydrolysed. It may be desirable to supply partially hydrolysed proteins (degree of hydrolysis between 2 and 20%), for example for infants or young children believed to be at risk of developing cow's milk allergy. If hydrolysed proteins are required, the hydrolysis process may be carried out as desired and as is known in the art. For example, whey protein hydrolysates may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

In an embodiment of the invention at least 70% of the proteins are hydrolysed, preferably at least 80% of the proteins are hydrolysed, such as at least 85% of the proteins are hydrolysed, even more preferably at least 90% of the proteins are hydrolysed, such as at least 95% of the proteins are hydrolysed, particularly at least 98% of the proteins are hydrolysed. In a particular embodiment, 100% of the proteins are hydrolysed.

In one particular embodiment the proteins of the nutritional composition are hydrolyzed, fully hydrolyzed or partially hydrolyzed. The degree of hydrolysis (DH) of the protein can be between 8 and 40, or between 20 and 60 or between 20 and 80 or more than 10, 20, 40, 60, 80 or 90.

The protein component can alternatively be replaced by a mixture of free amino acid, for example for preterm or low birth weight infants. The free amino acids can be obtained by complete hydrolysis of proteins (DH of 100) or can be synthetic amino acids.

In a particular embodiment the nutritional composition according to the invention is a hypoallergenic composition. In another particular embodiment the composition according to the invention is a hypoallergenic nutritional composition.

### Vitamins and minerals

The nutritional composition of the invention may also contain all vitamins and minerals understood to be essential in the daily diet and in nutritionally significant amounts. Minimum requirements have been established for certain vitamins and minerals. Examples of minerals, vitamins and other nutrients optionally present in the composition of the invention include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L-carnitine. Minerals are usually added in salt form. The presence and amounts of specific minerals and other vitamins will vary depending on the intended population.

### Other nutrients:

If necessary, the nutritional composition of the invention may contain emulsifiers and stabilisers such as soy, lecithin, citric acid esters of mono- and diglycerides, and the like.

The nutritional composition of the invention may also contain other substances which may have a beneficial effect such as lactoferrin, nucleotides, nucleosides, and the like.

The nutritional composition of the invention may also contain carotenoid(s). In some particular embodiments of the invention, the nutritional composition of the invention does not comprise any carotenoid.

### Other oligosaccharides

The nutritional composition according to the present invention may also comprise at least another oligosaccharide(s) (i.e. other than the fucosylated oligosaccharide(s) necessarily present in the composition) and/or at least a fiber(s) and/or at least a precursor(s) thereof. The other oligosaccharide and/or fiber and/or precursor thereof may be selected from the list comprising N-acetylated oligosaccharides, (other) fucosylated oligosaccharides, galacto-oligosaccharides (GOS), fructo-oligosaccharides (FOS), inulin, xylooligosaccharides (XOS), polydextrose, sialylated oligosaccharides, sialic acid, fucose and any combination thereof. They may be in an amount between 0 and 10% by weight of composition. In a particular embodiment, the nutritional composition can also contain at least one BMO (bovine milk oligosaccharide).

Suitable commercial products that can be used in addition to the oligosaccharides comprised in the oligosaccharide mixture to prepare the nutritional compositions according to the invention include combinations of FOS with inulin such as the product sold by BENEO under the trademark Orafti, or polydextrose sold by Tate & Lyle under the trademark STA-LITE^{®}.

### Sialylated oligosaccharide

In another particular embodiment, the nutritional composition according to the invention can comprise sialylated oligosaccharide(s). There can be one or several sialylated oligosaccharide(s). The sialylated oligosaccharide(s) can be selected from the group comprising 3' sialyllactose (3-SL), 6' sialyllactose (6-SL), and any combination thereof. In some embodiments of the invention the composition comprises 3-SL and 6-SL. In some particular embodiments the ratio between 3'-sialyllactose (3-SL) and 6'-sialyllactose (6-SL) can be in the range between 5:1 and 1:10, or from 3:1 and 1:1, or from 1:1 to 1:10. In some specific embodiments the sialylated oligosaccharide of the composition is 6' sialyllactose (6-SL).

The sialylated oligosaccharide(s) may be isolated by chromatographic or filtration technology from a natural source such as animal milks. Alternatively, they may be produced by biotechnological means using specific sialyltransferases or sialyldases, neuraminidases, either by an enzyme based fermentation technology (recombinant or natural enzymes), by chemical synthesis or by a microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP), from DP=1 onwards. Alternatively, sialyllactoses may be produced by chemical synthesis from lactose and free N'-acetylneuraminic acid (sialic acid). Sialyllactoses are also commercially available for example from Kyowa Hakko Kogyo of Japan.

In another preferred embodiment of the invention the nutritional composition may comprise from 0.005-5 g/L of sialylated oligosaccharides, or 0.008-2.5 g/L, or 0.01-1 g/L, or 0.02-0.7 g/L, for example 0.03-0.5 g/L.

The nutritional composition according to the invention can contain 0.004-3.8 g of sialylated oligosaccharides per 100g of composition on a dry weight basis, e.g. 0.006-1.9 g or 0.008-0.8 g or 0.015-0.5 g, for example 0.023-0.4 of sialylated oligosaccharides per 100g of composition on a dry weight basis.

In some particular embodiments of the present invention, the nutritional composition comprises sialylated oligosaccharide(s) in an amount of below 0.1g/100 g of composition on a dry weight basis.

In a particular embodiment, the sialylated oligosaccharide is provided in the nutritional composition of the present invention in such an amount that normal consumption of the nutritional composition or would provide to the infant or young child, respectively the child, consuming it a total daily dose of 0.003-6.5 g, preferably 0.005-3.3 g or 0.006-1.3 g or 0.01-0.9 g, for example 0.018-0.65 g per day.

In some particular embodiments of the present invention, the nutritional composition does not contain any sialylated oligosaccharide(s).

The nutritional composition according to the present invention may optionally also comprise at least one precursor of oligosaccharide. There can be one or several precursor(s) of oligosaccharide. For example the precursor of human milk oligosaccharide is sialic acid, fucose or a mixture thereof. In some particular embodiments the composition comprises sialic acid.

In particular examples the nutritional composition comprises from 0 to 3 g/L of precursor(s) of oligosaccharide, or from 0 to 2 g/L, or from 0 to 1 g/L, or from 0 to 0.7 g/L, or from 0 to 0.5 g/L or from 0 to 0.3 g/L, or from 0 to 0.2 g/L of precursor(s) of oligosaccharide. The composition according to the invention can contain from 0 to 2.1 g of precursor(s) of oligosaccharide per 100g of composition on a dry weight basis, e.g. from 0 to 1.5 g or from 0 to 0.8 g or from 0 to 0.15 g of precursor(s) of oligosaccharide per 100g of composition on a dry weight basis.

### Fucosylated oligosaccharide

The nutritional composition of the present invention contains at least one human milk oligosaccharides. It comprises at least one fucosylated oligosaccharide. There can be one or several types of fucosylated oligosaccharide(s). The fucosylated oligosaccharide(s) can indeed be selected from the list comprising 2'-fucosyllactose, 3'fucosyllactose, difucosyllactose, lacto-N-fucopentaose (such as lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose (such as fucosyllacto-N-neohexaose I, fucosyllacto-N-neohexaose II), difucosyllacto-N-hexaose I, difuco-lacto-N-neohexaose, difucosyllacto-N-neohexaose I, difucosyllacto-N-neohexaose II, fucosyl-para-Lacto-N-hexaose, tri-fuco-para-Lacto-N-hexaose I and any combination thereof.

In some particular embodiments the fucosylated oligosaccharide comprises a 2'-fucosyl-epitope. It can be for example selected from the list comprising 2'-fucosyllactose, difucosyllactose, lacto-N-fucopentaose, lacto-N-fucohexaose, lacto-N-difucohexaose, fucosyllacto-N-hexaose, fucosyllacto-N-neohexaose, difucosyllacto-N-hexaose difuco-lacto-N-neohexaose, difucosyllacto-N-neohexaose, fucosyl-para-Lacto-N-hexaose and any combination thereof.

In a particular preferable embodiment, the nutritional composition according to the invention comprises 2'-fucosyllactose (or 2FL, or 2'FL, or 2-FL or 2'-FL). It is believed that 2FL is the optimum fucosylated oligosaccharide in the context of the present invention, which it naturally contained in human Breast Milk is relatively significant amount.

In a particular embodiment, there is no other type of fucosylated oligosaccharide than 2'-fucosyllactose, i.e. the nutritional composition of the invention comprises only 2'-fucosyllactose as fucosylated oligosaccharide.

The fucosylated oligosaccharide(s) may be isolated by chromatography or filtration technology from a natural source such as animal milks. Alternatively, it may be produced by biotechnological means using specific fucosyltransferases and/or fucosidases either through the use of enzyme-based fermentation technology (recombinant or natural enzymes) or microbial fermentation technology. In the latter case, microbes may either express their natural enzymes and substrates or may be engineered to produce respective substrates and enzymes. Single microbial cultures and/or mixed cultures may be used. Fucosylated oligosaccharide formation can be initiated by acceptor substrates starting from any degree of polymerization (DP), from DP = 1 onwards. Alternatively, fucosylated oligosaccharides may be produced by chemical synthesis from lactose and free fucose. Fucosylated oligosaccharides are also available for example from Kyowa, Hakko, Kogyo of Japan.

The fucosylated oligosaccharide(s) may be present in the nutritional composition according to the invention in a total amount of 0.75-1.65 g/L of the composition. In some embodiments, the fucosylated oligosaccharide(s) may be in a total amount of 0.005-5 g/L of the composition, such as 0.01-3 g/L or 0.02-2 g/L or 0.1-2.5 g/L or 0.15-2 g/L or 0.25-1.9 g/L of the composition. In a particular embodiment, the fucosylated oligosaccharide(s) is/are in a total amount of 1 g/L of the composition. In another particular embodiment, the fucosylated oligosaccharide(s) is/are in a total amount of 0.25 or 0.26 g/L of the composition. In one embodiment the fucosylated oligosaccharide(s) is/are in a total amount of more than 0.1g/L and optionally less than 1g/L or more than 0.2 and less than 0.8g/L. In one embodiment the fucosylated oligosaccharide(s) is/are in a total amount of at least 0.1g/L, at least 0.25, at least 0.26, at least 0.5, at least 0.7, at least 0.8, at least 1, at least 1.25, at least 1.5 at least 1.5 or at least 2 g/L.

The fucosylated oligosaccharide(s) can be present in the nutritional composition in a total amount of 0.004-3.8 g/100g of composition on a dry weight basis. The fucosylated oligosaccharide(s) may be in a total amount of 0.008-2.3 g/100g of the composition, such as 0.015-1.5 g/100g, or 0.08-1.9 g/100g or 0.12-1.5 g/100g or 0.15-1.5 g/100g or 0.19-1.5 g/100g of the composition. In a particular embodiment, the fucosylated oligosaccharide(s) is/are in a total amount of 0.075 or 0.78 g/100g of the composition. In another particular embodiment, the fucosylated oligosaccharide(s) is/are in a total amount of 0.2 g/100g of the composition. In a particular embodiment, the fucosylated oligosaccharide(s) is/are in a total amount of at least 0.01g/100g, at least 0.02g/100g, at least 0.05g/100g, at least 0.1g/100g, at least 0.2g/100g, at least 0.25g/100g, at least 0.4g/100g, at least 0.5 g/100g, at least 0.75g/100g, at least 0.9g/100g, at least 1g/100g, at least 1.5g/100g, at least 2g/100g or at least 3g/100g of the composition.

In a particular embodiment, the fucosylated oligosaccharide is provided in the nutritional composition of the present invention in such an amount that normal consumption of the nutritional composition would provide to the infant or young child, respectively the child, consuming it a total daily dose of 0.003-6.5 g, preferably 0.006-3.9 g, for example 0.012-2.6 g per day. It is believed that a minimal amount of fucosylated oligosaccharide is necessary to have the desired effect in a measurable way.

For preterm, low birth weight and small for gestational age infants, the daily dose of fucosylated oligosaccharides is preferably of 0.05 to 1 g/kg of body weight per day, preferably 0.06-0.9 g or 0.07-0.8 g or 0.08-0.7 g or 0.09-0.6 g or 0.1-0.5 g or 0.2-0.4 g/, most preferably 0.34 g per kg of body weight and per day.

When both fucosylated and N-acetylated oligosaccharides are present, the fucosylated oligosaccharide(s) and the N-acetylated oligosaccharide(s) comprised in the nutritional composition according to the invention are typically present in a N-acetylated oligosaccharide(s):fucosylated oligosaccharide(s) of from 1:20 to 2:1, preferably 1:15 to 1:1, most preferably of 1:10 to 1:2. In a particularly advantageous embodiment, this ratio is (or is around) 1:2,1:5, or 1:10.

In a particular embodiment of the present invention, the nutritional composition comprises 2'-fucosyllactose (2FL) and no other oligosaccharide. In separate embodiment of the present invention, the nutritional composition comprises 2'-fucosyllactose (2FL) and another oligosaccharide, preferably a human milk oligosaccharide, more preferably lacto-N-neotetraose (LNnT) or LNT (lacto-N-tetraose).

In a particular embodiment of the present invention, the nutritional composition comprises 2'-fucosyllactose (2FL) and lacto-N-neotetraose (LNnT).

In specific embodiment, the nutritional composition of the present invention comprises an oligosaccharide mixture that consists of 2'-fucosyllactose (2-FL) and lacto-N-neotetraose (LNnT). In other words, the nutritional composition of the invention comprises only 2'-fucosyllactose (2-FL) as fucosylated oligosaccharide and only lacto-N-neotetraose (LNnT) as N-acetylated oligosaccharide.

### N-acetylated oligosaccharide(s)

In one embodiment the composition of the invention comprises at least one N-acetylated oligosaccharide. The N-acetylated oligosaccharide is preferably LnNT. The inventors has found, without being bound by the theory, that LnNT develops the best interactions with the gut flora and boost the described effect.

In one embodiment the N-acetylated oligosaccharide is lacto-N-neotetraose (LNnT), lacto-N-tetraose (LNT), para-lacto-N-neohexaose (para-LNnH), disialyllacto-N-tetraose (DSLNT) or any combination thereof.

In one embodiment the N-acetylated oligosaccharide(s) is present in a total amount of between 0.025-1.5 g/L of the composition, preferably at least 0.1g/L or at least 0.25g/L, and/or in a total amount of between 0.003-0.23 g/100g, preferably at least 0.015g/100g or at least 0.03g/100g of composition on a dry weight basis.

The N-acetylated oligosaccharide(s) may be synthesised chemically by enzymatic transfer of saccharide units from donor moieties to acceptor moieties using glycosyltransferases as described for example in US patent No. 5,288,637 and WO 96/10086. Alternatively, LNT and LNnT may be prepared by chemical conversion of Keto-hexoses (e.g. fructose) either free or bound to an oligosaccharide (e.g. lactulose) into N-acetylhexosamine or an N-acetylhexosamine-containing oligosaccharide as described in Wrodnigg, T.M.; Stutz, A.E. (1999) Angew. Chem. Int. Ed. 38:827-828. N-acetyl-lactosamine produced in this way may then be transferred to lactose as the acceptor moiety.

In a particularly advantageous embodiment of the present invention, the N-acetylated oligosaccharide(s) are present in the nutritional composition in some particular amounts. The term "amount" refers to the total amount of each of these two components in the nutritional composition unless otherwise specified. It therefore does not refer to an individual amount except when there is a single type of these components (in that case both the total and individual amounts equal). The same applies for all compounds/ingredients of the invention. By way of illustrative example, if there is only one (i.e. only one type of) N-acetylated oligosaccharide in the composition (e.g. LNnT), its individual amount (and therefore the total amount of N-acetylated oligosaccharides) will be in the range 0.75-1.65 g/L. If there are several (i.e. several types of) N-acetylated oligosaccharides, their individual amount will be lower (e.g. if there are 2 different types of N-acetylated oligosaccharide, e.g. LNnT + LNT, there may be for example each in an individual amount of 0.5 g/L) but the total amount of N-acetylated oligosaccharides will be in the range 0.75-1.65 g/L.

In a particular embodiment, the N-acetylated oligosaccharide is provided in the nutritional composition of the present invention in such an amount that normal consumption of the nutritional composition would provide to the infant or young child, respectively the child, consuming it a total daily dose of 0.003-3.9 g, preferably 0.006-3.25 g or 0.03-1.95 g or 0.03-1.3 g or 0.03-1 g, for example 0.05-1 g per day.

For preterm, low birth weight and small for gestational age infants, the daily dose is preferably of 0.005 to 0.1 g/kg of body weight per day, preferably 0.006-0.09 g or 0.007-0.08 g or 0.008-0.07 g or 0.009-0.06 g or 0.01-0.05 g or 0.02-0.04 g/, most preferably 0.034 g per kg of body weight and per day.

### Use and effect of the composition

A first object of the present invention is therefore a nutritional composition comprising at least one fucosylated oligosaccharide, optionally also at least one N-acetylated oligosaccharide, for use in enhancing the pancreatic development and/or the pancreatic maturation of infants. It is believed that the pancreatic development and pancreatic maturation are very much related and linked as the functionality of biosynthesis, storage and excretion (or active secretion) of hormones and enzymes by the mature organ depends on its development.

In one embodiment the nutritional composition comprises at least one fucosylated oligosaccharide is present in a total amount of between 0.05-3 g/L of the composition, preferably at least 0.2g/L or at least 0.5g/L, and/or in a total amount of between 0.007-0.45 g/100g, preferably at least 0.03g/100g or at least 0.075g/100g of composition on a dry weight basis.

### Target age

In one embodiment the nutritional composition of the invention is especially intended and designed for infants are between 0 and 12 months, preferably between 0 and 6 months. It is believed that an intervention at early age (below 6 or below 12 months of age) has a greater potential to have an effect. In one embodiment the invention is used for young children (toddlers) up to the age of 3 years. Without being bound by the theory the inventors believe that the effect of the invention can be positively expected not only for infants, but also for young children up to the age of 3 years (based on the corresponding age of the rats extrapolated to infants/young children in the experiments herein described).

### Effect

The invention pertains to the subject-matter of the claims.

In one embodiment the nutritional composition induces the enhancement of pancreatic development or maturation by inducing or mediated or being accompanied by the enhancement or increase of the level of biosynthesis of insulin by the pancreatic cells of the infant. By "enhancement" it is understood that the level of biosynthesis of insulin is increased (compared to subject not receiving the nutritional composition of the invention). The enhancement can interrelate with the increase of the number of (insulin producing) pancreatic β-cells, and/or their ability to synthetize insulin (in high amount) and/or more generally their degree of maturation. Importantly, such increased biosynthesis may or may not be accompanied by an increase in the secretion or excretion of insulin by the pancreas. Without being bound by the theory it is indeed believed that the biosynthesis and the secretion of insulin are regulated by different effectors. Also it is believed that increasing the level of biosynthesis by the pancreatic beta-cells provides an enhanced health effect mid and long term (compared to increasing the secretion) as the secretion (without increased biosynthesis) reaches a plateau very rapidly ("empty reservoir effect").

The inventors conclude that such improved maturation of the pancreatic beta-cells and such increased biosynthesis helps improving the glucose management of the infants during the administration period and/or during infancy and/or later in life. Related negative health conditions such as risk of diabetes (type-2), obesity and/or over-weight are therefore reduced, and prevented.

Without to be bound by the theory, the inventors believe that the increased biosynthesis of insulin by the pancreatic cells is a better indicator of short and long term health benefits, compared to the promotion of insulin secretion. Promoting merely the insulin secretion cannot have the same optimum benefit as those are limited by the plateau effect corresponding to the limiting amount of insulin biosynthesized by the pancreatic cells (secretion can only make available the amount biosynthesized; while increased biosynthesis inevitably leads to high amount of circulating insulin longer term).

The infants are subject in need and/or are at risk of under-development of pancreas and/or are born preterm and/or suffering from intra-uterine-growth retardation (IUGR) and/or born with low birth weight (LBW), very low birth weight (VLBW), or extremely-low-birth-weight (ELBW). Without being bound by the theory, the inventors believe that the more fragile the infants are (e.g. the lower the birth-weight, or the more premature they are), the more the infants can benefit from the invention. Indeed it is believed that those infants fail to produce (or are at risk of failing to produce) the optimal amount of insulin (due to immaturity of their pancreatic function). Such sub-normal pancreatic functionality is extremely difficult to regulate but can induce multiple glucose-management associated pathologies. Among those, short term, high blood glucose / low insulin plasma can be observed. Long term, it is associated with diabetes (type 2), risk of obesity and/or over-weight and all the related cardiovascular diseases. The inventor has found that the proposed nutritional intervention can lead to a faster / more optimum establishment of the pancreatic functions, in particular in those infants at risk, and a reduction of the risk of the associated diseases or pathological conditions (both long term and short term).

### Preparation of the composition

The nutritional composition according to the invention may be prepared in any suitable manner. A composition will now be described by way of example.

For example, a formula such as an infant formula may be prepared by blending together the protein source, the carbohydrate source and the fat source in appropriate proportions. If used, the emulsifiers may be included at this point. The vitamins and minerals may be added at this point but they are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently in the range between about 50°C and about 80°C to aid dispersal of the ingredients. Commercially available liquefiers may be used to form the liquid mixture. The fucosylated oligosaccharide(s) and/or the N-acetylated oligosaccharide(s) may be added at this stage, especially if the final product is to have a liquid form. If the final product is to be a powder, they may likewise be added at this stage if desired.

The liquid mixture is then homogenised, for example in two stages.

The liquid mixture may then be thermally treated to reduce bacterial loads, by rapidly heating the liquid mixture to a temperature in the range between about 80°C and about 150°C for a duration between about 5 seconds and about 5 minutes, for example. This may be carried out by means of steam injection, an autoclave or a heat exchanger, for example a plate heat exchanger.

Then, the liquid mixture may be cooled to between about 60°C and about 85°C for example by flash cooling. The liquid mixture may then be again homogenised, for example in two stages between about 10 MPa and about 30 MPa in the first stage and between about 2 MPa and about 10 MPa in the second stage. The homogenised mixture may then be further cooled to add any heat sensitive components, such as vitamins and minerals. The pH and solids content of the homogenised mixture are conveniently adjusted at this point.

If the final product is to be a powder, the homogenised mixture is transferred to a suitable drying apparatus such as a spray dryer or freeze dryer and converted to powder. The powder should have a moisture content of less than about 5% by weight. The fucosylated oligosaccharide(s) and the N-acetylated oligosaccharide(s) may also or alternatively be added at this stage by dry-mixing or by blending them in a syrup form of crystals, along with the probiotic strain(s) (if used), and the mixture is spray-dried or freeze-dried.

If a liquid composition is preferred, the homogenised mixture may be sterilised then aseptically filled into suitable containers or may be first filled into the containers and then retorted.

### Target subjects for the composition

The nutritional composition of the present invention may also be used in an infant or a young child that was born by C-section or that was vaginally delivered.

The nutritional composition according to the invention is for use in infants or young children. The infants or young children may be born term or preterm. In a particular embodiment the nutritional composition of the invention is for use in infants or young children that were born preterm, having a low birth weight and/or born small for gestational age (SGA). In a particular embodiment the nutritional composition of the invention is for use in preterm infants, infants having a low birth weight and/or infants born small for gestational age (SGA). It is believed that infant born preterm or born with low birth weight achieved necessarily a lower stage of development *in utero* and thus can benefit from the nutritional intervention of the invention.

Infants in needs of optimal pancreatic development or maturation are particular targets for the invention. Such infants may exhibit a risk, or a measurable, of under-development or under-maturation of the pancreas at birth. Consequently such infants can best benefit of the subject invention. The invention helps the subject in needs to compensate the under-development or under-maturation of their pancreatic functionality by the promotion (induce by the nutritional intervention of the invention) of a faster post-birth maturation/development and thus promote an optimum functionality of the pancreatic cells, in particular promote the biosynthesis of insulin.

Preterm infants (infant born before the normal term), low-birth weight infants or growth-retarded infants are particular target for the invention. Generally the more preterm or low birth weight the infants are, the highest the benefits of the invention on their short term and long term glucose management.

Any reference to prior art documents in this specification is not to be considered an admission that such prior art is widely known or forms part of the common general knowledge in the field.

The invention will now be described in further details. It is noted that the various aspects, features, examples and embodiments described in the present application may be compatible and/or combined together.

### Examples

The following examples illustrate some specific embodiments of the composition for use according to the present invention. The examples are given solely for the purpose of illustration and are not to be construed as limitations of the present invention.

### Example 1

An example of the composition of a nutritional composition (e.g. an infant formula) according to the present invention is given in the below table 1. This composition is given by way of illustration only.

**Table 1: Composition of the infant formula of Example 1**

| **Nutrients** | | **per 100kcal** | **per litre** |
|---|---|---|---|
| Energy (kcal) | | 100 | 670 |
| Protein (g) | | 1.83 | 12.3 |
| Fat (g) | | 5.3 | 35.7 |
| Linoleic acid (g) | | 0.79 | 5.3 |
| α-Linolenic acid (mg) | | 101 | 675 |
| Lactose (g) | | 11.2 | 74.7 |
| Minerals (g) | | 0.37 | 2.5 |
| Na (mg) | | 23 | 150 |
| K (mg) | | 89 | 590 |
| Cl (mg) | | 64 | 430 |
| Ca (mg) | | 62 | 410 |
| P (mg) | | 31 | 210 |
| Mg (mg) | | 7 | 50 |
| Mn (µg) | | 8 | 50 |
| Se (µg) | | 2 | 13 |
| Vitamin A (µg RE) | | 105 | 700 |
| Vitamin D (µg) | | 1.5 | 10 |
| Vitamin E (mg TE) | | 0.8 | 5.4 |
| Vitamin K1 (µg) | | 8 | 54 |
| Vitamin C (mg) | | 10 | 67 |
| Vitamin B1 (mg) | | 0.07 | 0.47 |
| Vitamin B2 (mg) | | 0.15 | 1.0 |
| Niacin (mg) | | 1 | 6.7 |
| Vitamin B6 (mg) | | 0.075 | 0.50 |
| Folic acid (µg) | | 9 | 60 |
| Pantothenic acid (mg) | | 0.45 | 3 |
| Vitamin B12 (µg) | | 0.3 | 2 |
| Biotin (µg) | | 2.2 | 15 |
| Choline (mg) | | 10 | 67 |
| Fe (mg) | | 1.2 | 8 |
| I (µg) | | 15 | 100 |
| Cu (mg) | | 0.06 | 0.4 |
| Zn (mg) | | 0.75 | 5 |
| Oligosaccharides (HMOs) | 2FL (g) | 0.075 | 0.5 |

### Example 2:

An example of the composition of a nutritional composition (e.g. an infant formula) according to the present invention is given in the below table 2. This composition is given by way of illustration only.

**Table 2: Composition of the infant formula of Example 2**

| **Nutrients** | | **per 100kcal** | **per litre** |
|---|---|---|---|
| Energy (kcal) | | 100 | 670 |
| Protein (g) | | 1.83 | 12.3 |
| Fat (g) | | 5.3 | 35.7 |
| Linoleic acid (g) | | 0.79 | 5.3 |
| α-Linolenic acid (mg) | | 101 | 675 |
| Lactose (g) | | 11.2 | 74.7 |
| Minerals (g) | | 0.37 | 2.5 |
| Na (mg) | | 23 | 150 |
| K (mg) | | 89 | 590 |
| Cl (mg) | | 64 | 430 |
| Ca (mg) | | 62 | 410 |
| P (mg) | | 31 | 210 |
| Mg (mg) | | 7 | 50 |
| Mn (µg) | | 8 | 50 |
| Se (µg) | | 2 | 13 |
| Vitamin A (µg RE) | | 105 | 700 |
| Vitamin D (µg) | | 1.5 | 10 |
| Vitamin E (mg TE) | | 0.8 | 5.4 |
| Vitamin K1 (µg) | | 8 | 54 |
| Vitamin C (mg) | | 10 | 67 |
| Vitamin B1 (mg) | | 0.07 | 0.47 |
| Vitamin B2 (mg) | | 0.15 | 1.0 |
| Niacin (mg) | | 1 | 6.7 |
| Vitamin B6 (mg) | | 0.075 | 0.50 |
| Folic acid (µg) | | 9 | 60 |
| Pantothenic acid (mg) | | 0.45 | 3 |
| Vitamin B12 (µg) | | 0.3 | 2 |
| Biotin (µg) | | 2.2 | 15 |
| Choline (mg) | | 10 | 67 |
| Fe (mg) | | 1.2 | 8 |
| I (µg) | | 15 | 100 |
| Cu (mg) | | 0.06 | 0.4 |
| Zn (mg) | | 0.75 | 5 |
| Oligosaccharides (HMOs) | 2FL (g) | 0.15 | 1 |
| | LNnT (g) | 0.075 | 0.5 |

### Example 3

### Description of the study

Three groups of time-mated pregnant Sprague-Dawley female rats were bought from Charles River laboratories. One group was submitted to food restriction of 60% during the last 10 days of gestation and their offspring were cross-fostered to normally fed rats. A second group of pregnant females was normally fed and their offspring cross-fostered among the same group of dams. Immediately after birth (postnatal day 2 (d=2)), the born rat pups - subjects of the experiment - were assigned to one of the following groups:
- IUGR group (negative control; n=20): IUGR rats being fed a normal diet after birth;
- IUGR rats + HMO (test groups): During the experiment, they were reared by their mothers for 21 days and supplemented with HMOs (2-FL, LNnT or the Mix of 2-FL and LNnT). At weaning, they were fed a diet supplemented with the same HMOs, see details below. There were three different groups:
   - IUGR/2FL group (n=10): supplemented with 2-FL only;
   - IUGR/LNnT group (n=10): supplemented with LNnT only; and
   - IUGR/HMOs mix group (n=10): supplemented with LNnT and 2FL in a weight ratio of 1:2.

All rat pups were fed from birth (d =1) up to 57 days (d = 57) based on the following protocol:
- d1 to d6: all groups of rats were nursed by dam (mothers' milk only);
- d7 to d21: all rats were nursed by dam (mothers' milk only). But they were also supplemented with 3 g/ kg body weight of HMOs in the test groups or maltodextrin for the control group via gavage:
   - 3 g/ kg body weight of 2-FL, for the IUGR/2FL group;
   - 3 g/ kg body weight of LNnT, for the IUGR/LNnT group;
   - 3 g/kg body weight of a mix of LNnT and 2FL at a 1:2 weight ratio, for the IUGR/HMOs mix group;
- d22 to d57: all rats were fed a diet as detailed in Table 3 below.

**Table 3: Composition of diets fed to the different groups from d22 to d57**

| | Amount in diet of Control Group [%] | Amount in diet of 2FL Group [%] | Amount in diet of LNnT Group [%] | Amount in diet of HMO Mix Group [%] |
|---|---|---|---|---|
| Cornstarch | 53.4 | 53.4 | 53.4 | 53.4 |
| Casein | 20 | 20 | 20 | 20 |
| Sucrose | 10 | 10 | 10 | 10 |
| Soybean oil | 7 | 7 | 7 | 7 |
| Mineral Mix AIN-93-G * | 3.5 | 3.5 | 3.5 | 3.5 |
| Choline bitartrate | 0.25 | 0.25 | 0.25 | 0.25 |
| L-Cystine | 0.3 | 0.3 | 0.3 | 0.3 |
| Tert-butylhydroquinone | 0.0014 | 0.0014 | 0.0014 | 0.0014 |
| Vitamin Mix AIN-93-VX * | 1 | 1 | 1 | 1 |
| Maltodextrin | 4.5 | 0 | 0 | 0 |
| 2FL | 0 | 4.5 | 0 | 3 |
| LNnT | 0 | 0 | 4.5 | 1.5 |

| | | | | |
|---|---|---|---|---|
| * from Research Diets, Inc. | | | | |

Rats were sacrificed at day 57 (postnatal day 57 = p57) after a 6-hr fasting period and pancreas was dissected and its insulin content was extracted using a solution acid-ethanol and quantified using an Ultra Sensitive Insulin ELISA kit (Crystallnc., Downer Grove, IL, USA) with and inter assay CV of 4.0%.

### Findings

After sacrifice (day 57 = p57) the insulin content of the rat's pancreas was analysed and is reported in Figure 1. The concentration is expressed as amount of insulin (in µg) per gram of pancreatic tissue-. The control IUGR rats express an insulin value of about 50µg insulin per g pancreatic tissue. The rat group fed LNnT also expressed about the same level of insulin.

The rats fed with 2FL surprisingly expressed a significantly higher value of insulin in their pancreatic tissues. Even more surprisingly, the rats fed with a mix of 2FL and LNnT also expressed a high value of insulin in their pancreatic tissues.

The inventors concluded that the diet with 2FL in the specific population enhances the development and/or maturation of the pancreatic cells, thus enhancing the biosynthesis of insulin. The invention also conclude that not all HMOs have a similar effect (LNnT alone for example does not seems to boost the insulin biosynthesis). Amazingly the combination of 2FL and LNnT in the diet also boosts the biosynthesis of insulin - tendntially in a synergistic way compared to 2FL alone (or LNnT alone). At the very least the presence of LNnT does not inhibit the insulin-synthesis boosting effect of 2FL in the diet. Without being bound by the theory, this may indicate that each HMO (2FL, LNnT) leverage a different mechanism leading to the enhancement of the biosynthesis of insulin. Altogether these results show that 2FL alone or in combination with LNnT was successful in increasing the biosynthesis of insulin.

By selecting the most proper HMO and feeding the subject with the selected HMO, the inventors hence arrived to the enhancement of the maturation of pancreas and consequently to the boost of the insulin secretion.

It shows that a nutritional intervention with the selected bioactive nutrient can induce a measurable change in the pancreatic insulin content during the phase of pancreatic development and it can, as such influence the pancreatic maturation. Based on these observations, it is highly probable that the long term effect can be envisioned in such conditions depending on pancreatic maturation and/or level of insulin biosynthesis.

### Example 4

A supplement for preterm infants is prepared, such as to provide the following daily dose:
- 0.34 g/kg of body weight per day of 2-FL, and
- optionally 0.034g/kg of body weight per day of LNnT;

## Claims

1. A nutritional composition comprising at least one fucosylated oligosaccharide for use in enhancing the pancreatic development and/or pancreatic maturation of infants or young children, wherein said infants or young children are subject in need and/or are at risk of under-development of pancreas and/or are born preterm and/or suffering from intra-uterine-growth retardation (IUGR) and/or born with low birth weight (LBW), very-low-birth-weight (VLBW), or extremely-low-birth-weight (ELBW).

2. The nutritional composition for use of claim 1 wherein said at least one fucosylated oligosaccharide is present in a total amount of between 0.05-3 g/L of the composition and/or in a total amount of between 0.007-0.45 g/100g of composition on a dry weight basis.

3. The nutritional composition for use of any of the preceding claims wherein said fucosylated oligosaccharide is 2FL.

4. The nutritional composition for use of any of the preceding claims wherein said composition comprises a N-acetylated oligosaccharide.

5. The nutritional composition for use of any of the preceding claims wherein said N-acetylated oligosaccharide is lacto-N-neotetraose (LNnT), lacto-N-tetraose (LNT), para-lacto-N-neohexaose (para-LNnH), disialyllacto-N-tetraose (DSLNT) or any combination thereof.

6. The nutritional composition for use of claim 4 or claim 5 wherein said N-acetylated oligosaccharide is present in a total amount of between 0.025-1.5 g/L of the composition and/or in a total amount of between 0.003-0.23 g/100g of composition on a dry weight basis.

7. The nutritional composition for use of any of the preceding claims wherein said nutritional composition is an Infant Formula, or a supplement for infants or young children, or a fortifier for Human Breast Milk, or a follow-on formula, or a Growing-Up Milk.

8. The nutritional composition for use of any of the preceding claims wherein said composition is to be administered during the first 1, 2, 3, 6, or 12 months of life, and/or administered in an amount and/or a duration sufficient to induce a measurable enhancement of the pancreatic development or pancreatic maturation of said infants.

9. The nutritional composition for use of any of the preceding claims wherein said composition further comprises Sialyllactose, preferably 3SL or 6SL.

10. The nutritional composition for use of any one of the preceding claims, comprising at least another oligosaccharide(s) and/or fiber(s) and/or precursor(s) thereof selected from the list comprising galacto-oligosaccharides (GOS), fructo-oligosaccharides (FOS), xylooligosaccharides (XOS), inulin, polydextrose, sialylated oligosaccharides, sialic acid, fucose and any combination thereof.

11. The nutritional composition for use of any one of the preceding claims, said composition further comprising at least one probiotic, live or in a non-replicating form, in an amount of from 10³ to 10¹² cfu/g of said composition (dry weight).

12. The nutritional composition for use of any one of claims 4 to 11 wherein said composition is an infant formula, said N-acetylated oligosaccharide is LNnT and said fucosylated oligosaccharide is 2FL, and wherein said infant is a preterm infant, optionally said composition being fed during the first 6 months of life to said infant.

## Patentansprüche

1. Ernährungszusammensetzung, umfassend mindestens ein fucosyliertes Oligosaccharid, zur Verwendung bei einem Fördern der Pankreasentwicklung und/oder Pankreasmaturation bei Säuglingen oder Kleinkindern, wobei die Säuglinge oder die Kleinkinder ein bedürftiges Subjekt sind und/oder für eine Unterentwicklung der Pankreas gefährdet sind und/oder vorzeitig geboren wurden und/oder an intrauteriner Wachstumsverzögerung (IUGR) leiden und/oder mit niedrigem Geburtsgewicht (LBW), sehr niedrigem Geburtsgewicht (VLBW) oder extrem niedrigem Geburtsgewicht (ELBW) geboren wurden.

2. Ernährungszusammensetzung zur Verwendung nach Anspruch 1, wobei das mindestens eine fucosylierte Oligosaccharid in einer Gesamtmenge zwischen 0,05-3 g/l der Zusammensetzung und/oder in einer Gesamtmenge zwischen 0,007-0,45 g/100 g der Zusammensetzung auf einer Trockengewichtsbasis vorhanden ist.

3. Ernährungszusammensetzung zur Verwendung nach einem der vorstehenden Ansprüche, wobei das fucosylierte Oligosaccharid 2FL ist.

4. Ernährungszusammensetzung zur Verwendung nach einem der vorstehenden Ansprüche, wobei die Zusammensetzung ein N-acetyliertes Oligosaccharid umfasst.

5. Ernährungszusammensetzung zur Verwendung nach einem der vorstehenden Ansprüche, wobei das N-acetylierte Oligosaccharid Lacto-N-neotetraose (LNnT), Lacto-N-tetraose (LNT), Para-lacto-N-neohexaose (Para-LNnH), Disialyllacto-N-tetraose (DSLNT) oder eine beliebige Kombination davon ist.

6. Ernährungszusammensetzung zur Verwendung nach Anspruch 4 oder 5, wobei das N-acetylierte Oligosaccharid in einer Gesamtmenge zwischen 0,025-1,5 g/l der Zusammensetzung und/oder in einer Gesamtmenge zwischen 0,003-0,23 g/100 g der Zusammensetzung auf einer Trockengewichtsbasis vorhanden ist.

7. Ernährungszusammensetzung zur Verwendung nach einem der vorstehenden Ansprüche, wobei die Ernährungszusammensetzung eine Säuglingsanfangsnahrung oder ein Nahrungsergänzungsmittel für Säuglinge oder Kleinkinder oder ein Stärkungsmittel für menschliche Muttermilch oder eine Folgenahrung oder eine Wachstumsmilch ist.

8. Ernährungszusammensetzung zur Verwendung nach einem der vorstehenden Ansprüche, wobei die Zusammensetzung während der ersten 1, 2, 3, 6 oder 12 Lebensmonate verabreicht werden soll und/oder in einer Menge und/oder für eine Dauer verabreicht werden soll, die ausreicht, um eine messbare Verbesserung der Pankreasentwicklung oder der Pankreasmaturation der Säuglinge zu bewirken.

9. Ernährungszusammensetzung zur Verwendung nach einem der vorstehenden Ansprüche, wobei die Zusammensetzung ferner Sialyllactose, vorzugsweise 3SL oder 6SL, umfasst.

10. Ernährungszusammensetzung zur Verwendung nach einem der vorstehenden Ansprüche, umfassend mindestens ein anderes Oligosaccharid(e) und/oder (eine) Faser(n) und/oder (einen) Vorläufer davon, ausgewählt aus der Liste, umfassend Galactooligosaccharide (GOS), Fructooligosaccharide (FOS), Xylooligosaccharide (XOS), Inulin, Polydextrose, sialylierte Oligosaccharide, Sialinsäure, Fucose und eine beliebige Kombination davon.

11. Ernährungszusammensetzung zur Verwendung nach einem der vorstehenden Ansprüche, die Zusammensetzung ferner umfassend mindestens ein Probiotikum, lebend oder in einer nicht replizierenden Form, in einer Menge von 10³ bis 10¹² KBE/g der Zusammensetzung (Trockengewicht).

12. Ernährungszusammensetzung zur Verwendung nach einem der Ansprüche 4 bis 11, wobei die Zusammensetzung eine Säuglingsanfangsnahrung ist, das N-acetylierte Oligosaccharid LNnT ist und das fucosylierte Oligosaccharid 2FL ist, und wobei der Säugling ein Frühgeborenes ist, wobei der Säugling optional während der ersten 6 Lebensmonate mit der Zusammensetzung gefüttert wird.

## Revendications

1. Composition nutritionnelle comprenant au moins un oligosaccharide fucosylé pour utilisation dans l'amélioration du développement pancréatique et/ou de la maturation pancréatique de nourrissons ou jeunes enfants, dans laquelle lesdits nourrissons ou jeunes enfants sont un sujet qui en a besoin et/ou sont à risque de sous-développement du pancréas et/ou sont nés prématurément et/ou souffrent d'un retard de croissance intra-utérin (IUGR) et/ou sont nés avec un faible poids à la naissance (LBW), un très faible poids à la naissance (VLBW) ou un poids à la naissance extrêmement faible (ELBW).

2. Composition nutritionnelle pour utilisation selon la revendication 1 dans laquelle ledit au moins un oligosaccharide fucosylé est présent en une quantité totale comprise entre 0,05 et 3 g/L de la composition et/ou en une quantité totale comprise entre 0,007 et 0,45 g/100 g de composition sur une base de poids sec.

3. Composition nutritionnelle pour utilisation selon l'une quelconque des revendications précédentes dans laquelle ledit oligosaccharide fucosylé est 2FL.

4. Composition nutritionnelle pour utilisation selon l'une quelconque des revendications précédentes dans laquelle ladite composition comprend un oligosaccharide N-acétylé.

5. Composition nutritionnelle pour utilisation selon l'une quelconque des revendications précédentes dans laquelle ledit oligosaccharide N-acétylé est lacto-N-néotétraose (LNnT), lacto-N-tétraose (LNT), para-lacto-N-néohexaose (para-LNnH), disialyllacto-N-tétraose (DSLNT) ou n'importe quelle combinaison de ceux-ci.

6. Composition nutritionnelle pour utilisation selon la revendication 4 ou la revendication 5 dans laquelle ledit oligosaccharide N-acétylé est présent en une quantité totale comprise entre 0,025 et 1,5 g/L de la composition et/ou en une quantité totale comprise entre 0,003 et 0,23 g/100 g de composition sur une base de poids sec.

7. Composition nutritionnelle pour utilisation selon l'une quelconque des revendications précédentes dans laquelle ladite composition nutritionnelle est une préparation pour nourrissons, ou un complément pour nourrissons ou jeunes enfants, ou un fortifiant pour lait maternel humain, ou une préparation de suite, ou un lait de croissance.

8. Composition nutritionnelle pour utilisation selon l'une quelconque des revendications précédentes dans laquelle ladite composition doit être administrée pendant les 1, 2, 3, 6 ou 12 premiers mois de vie, et/ou administrée en une quantité et/ou une durée suffisantes pour induire une amélioration mesurable du développement pancréatique ou de la maturation pancréatique desdits nourrissons.

9. Composition nutritionnelle pour utilisation selon l'une quelconque des revendications précédentes dans laquelle ladite composition comprend en outre du sialyllactose, de préférence 3SL ou 6SL.

10. Composition nutritionnelle pour utilisation selon l'une quelconque des revendications précédentes, comprenant au moins un ou plusieurs autre(s) oligosaccharide(s) et/ou fibre(s) et/ou précurseur(s) de ceux-ci choisis parmi la liste comprenant galacto-oligosaccharides (GOS), fructo-oligosaccharides (FOS), xylo-oligosaccharides (XOS), inuline, polydextrose, oligosaccharides sialylés, acide sialique, fucose et n'importe quelle combinaison de ceux-ci.

11. Composition nutritionnelle pour utilisation selon l'une quelconque des revendications précédentes, ladite composition comprenant en outre au moins un probiotique, vivant ou sous une forme non réplicative, en une quantité allant de 10³ à 10¹² ufc/g de ladite composition (poids sec).

12. Composition nutritionnelle pour utilisation selon l'une quelconque des revendications 4 à 11 dans laquelle ladite composition est une préparation pour nourrissons, ledit oligosaccharide N-acétylé est LNnT et ledit oligosaccharide fucosylé est 2FL, et dans laquelle ledit nourrisson est un nourrisson prématuré, facultativement ladite composition étant donnée pendant les 6 premiers mois de vie audit nourrisson.
